# NOUVEAU FASCICULE DE BREVET EUROPEEN

(11) **EP 2 449 985 B2**
(45) Date de publication et mention de la décision concernant l'opposition: **13.07.2022**
(45) Mention de la délivrance du brevet: 03.08.2016
(21) Numéro de dépôt: 11188050.6
(22) Date de dépôt: 07.11.2011
(51) Int. Cl.: A61B 17/16

(54) **Fraiseuse orthopédique de préparation osseuse, en particulier de préparation glénoïdienne**
Orthopädisches Frässwerkzeug zum Vorbereitung von Knochen, insbesondere zum Vorbereitung von Schultergelenkpfanne
Orthopaedic reamer for bone preparation, in particular for glenoïd preparation

(30) Priorité: 10.11.2010 FR 1059302; 08.11.2010 US 411429 P
(43) Date de publication de la demande: 09.05.2012
(73) Titulaire: Tornier, 38330 Montbonnot-Saint-Martin (FR)
(72) Inventeur: Purdy, Matthew J., Eden Prairie, Minnesota 55344 (US); Deransart, Pierric, 38410 Saint Martin D'Uriage (FR); Ferrand, Lucile, 38330 Montbonnot (FR)
(74) Mandataire: August Debouzy

(56) Documents cités:
- WO-A1-2005/094693
- WO-A1-2009/083707
- WO-A1-2011/012318
- DE-T2- 69 825 973
- GB-A- 2 406 278
- US-A- 3 559 514
- US-A- 5 203 653
- US-A1- 2003 163 135
- US-A1- 2004 236 339
- US-A1- 2006 015 110
- US-A1- 2006 058 809
- US-A1- 2007 038 302
- US-A1- 2007 038 303
- US-A1- 2007 276 393
- US-A1- 2009 270 863
- US-A1- 2013 150 859
- ZIMMER: "Zimmer Trabecular Metal Glenoid, Surgical Technique, Interference fit for secure initial fixation", , 2009, pages 1pp, 1-14, 3pp,
- DePuy Orthopaedics, Inc.: "GLOBAL ENABLE Glenoid Exposure Simplified, Design Rationale and Surgical Technique", , 2010, pages 2pp, 3-23, 1pp,

## Description

La présente invention concerne un ensemble de préparation osseuse, en particulier de préparation glénoïdienne, comportant une fraiseuse orthopédique.

Lors de l'implantation d'un composant glénoïdien d'une prothèse d'épaule, le chirurgien doit préparer la glène du patient opéré en vue d'y appuyer et d'y immobiliser ce composant. Pour ce faire, le chirurgien utilise généralement une fraiseuse à entraînement motorisé ou à entraînement manuel. Cette fraiseuse comprend ainsi une fraise dont le côté proximal est solidaire d'un manche d'entraînement, en rotation ou en oscillation autour de son axe central, tandis que la face distale de cette fraise est pourvue de reliefs, tels que des dents, des lames et/ou des pointes, qui permettent d'attaquer la matière osseuse constituant la glène, afin de la creuser, l'entamer et/ou la râper, jusqu'à donner à la glène une forme adaptée aux composants prothétiques glénoïdiens à implanter. Par exemple, la fraise creuse dans la glène une cavité sensiblement sphérique, dans laquelle viendra se loger de manière sensiblement complémentaire une partie dédiée du composant prothétique glénoïdien.

Par ailleurs, on sait qu'un des paramètres importants pour pérenniser la stabilité d'implantation d'un composant prothétique glénoïdien est lié au bon positionnement de ce composant sur la glène. Ainsi, lors des opérations de chirurgie visant à implanter un tel composant glénoïdien, il est répandu d'utiliser une broche de guidage orthopédique, consistant en une tige qui est enfoncée en partie dans la glène, avec précision en un point prédéterminé de celle-ci et selon une direction prédéterminée, de sorte que la partie longitudinale de cette tige qui demeure en saillie de la glène est ensuite utilisée par le chirurgien pour guider différents ancillaires manipulés par la suite, notamment des ancillaires de préparation osseuse de la glène, ces ancillaires étant engagés axialement autour de la broche de guidage, en étant enfilés le long de cette broche. En particulier, le chirurgien dispose de fraiseuses, telles que celles évoquées plus haut, dont la fraise inclut un moyeu dont l'alésage traversant central est dimensionné pour recevoir axialement la broche de guidage : lors de l'intervention chirurgicale, après avoir mis en place la broche de guidage, le chirurgien enfile axialement l'extrémité proximale de la broche dans l'alésage central de la fraise, puis fait glisser cette fraise et son manche proximal le long de la broche de guidage, en direction de la glène, jusqu'à plaquer la face distale de la fraise contre la glène. De telles fraises à moyeu central sont également désignées par l'expression « fraises canulées ». En entraînant alors la fraise en rotation ou en oscillation sur elle-même autour de la broche de guidage, la glène est fraisée avec précision, dans le sens où le fraisage réalisé est bien positionné vis-à-vis de la glène grâce à la coopération entre le moyeu central de la fraise et la broche du guidage.

Le document US-A-2006/015110 divulgue une fraise canulée telle qu'évoquée ci-dessus. A son extrémité distale, le moyeu de cette fraise délimite une fente dont l'étendue est très limitée suivant la direction axiale de l'alésage du moyeu : cette fente localisée à l'extrémité distale du moyeu permet de recevoir latéralement l'extrémité d'une broche de guidage orthopédique, de sorte que cette extrémité peut atteindre radialement l'alésage du moyeu, ce qui facilite quelque peu l'engagement du moyeu autour de la broche.

De son côté, WO-A-2005/094693, sur lequel est basé le préambule de la revendication 1, divulgue un ensemble de préparation osseuse comprenant une fraise canulée, telle qu'évoquée plus haut, et une broche de guidage spéciale : cette broche inclut, d'une part, une partie terminale distale, conçue, notamment de par sa rigidité qui est liée, entre autres, au dimensionnement ad hoc de sa section transversale, pour être enfoncée plus ou moins profondément dans un os à préparer, et, d'autre part, une partie terminale proximale, présentant une petite section transversale. L'enseignement de WO-A-2005/094693 repose sur le fait que la partie de broche à petite section, qui est prévue bien plus fine que la partie terminale distale de cette broche et qui peut même être prévue flexible, peut être engagée radialement jusqu'à l'intérieur du moyeu de la fraise canulée via une fente fine prévue tout le long du moyeu. Cependant, comme cela est d'ailleurs expliqué dans WO-A-2005/094693, on comprend que, alors que la partie terminale distale de la broche est enfoncée plus ou moins profondément dans un os à fraiser, il est indispensable, après que la partie de broche à petite section a été positionnée à l'intérieur du moyeu, de rapprocher ce moyeu en direction de l'os pour engager axialement dans ce moyeu l'extrémité, émergeant de l'os, de la partie terminale distale de la broche.

L'utilisation de fraises canulées, y compris celle de US-A-2006/015110, pose des difficultés de mise en oeuvre, liées à la présence des tissus mous autour de la glène à préparer, ainsi qu'à la proximité de la tête de l'humérus du patient opéré. Il s'avère donc nécessaire, à la fois, de mettre en place des écarteurs pour exposer largement la glène en vue de l'approcher selon la direction longitudinale de la broche, sans induire d'interférences entre les tissus mous et la fraise coulissée le long de la broche de guidage jusqu'à la glène, et de déplacer fortement la tête de l'humérus, voire de la réséquer. Le trauma et les cicatrices en résultant sont ainsi significatifs pour le patient.

Le but de la présente invention est de proposer une fraiseuse orthopédique dont l'utilisation soit plus facile pour le chirurgien et moins traumatique pour le patient.

A cet effet, l'invention a pour effet un ensemble de préparation osseuse, en particulier de préparation glénoïdienne, tel que défini à la revendication 1.

En lien avec cette fraiseuse, on divulgue également ici une méthode chirurgicale de préparation d'un os, en particulier d'une glène, par fraisage, dans laquelle :
- on expose un os à fraiser,
- on met en place dans cet os une broche de guidage orthopédique,
- on dispose d'une fraise qui inclut un moyeu muni d'un alésage qui est à même de recevoir la broche de guidage et qui délimite une fente de passage latéral pour la broche de guidage, cette fente débouchant de manière sensiblement radiale dans l'alésage et reliant les faces proximale et distale du moyeu sur lesquelles débouche l'alésage,
- on approche la fraise latéralement à la broche de guidage de manière qu'une partie courante de la broche de guidage soit engagée radialement à travers la fente jusqu'à atteindre l'intérieur de l'alésage, et
- on entraîne la fraise autour de la broche pour fraiser l'os.

L'idée à la base de l'invention est de chercher à approcher la fraise latéralement à la broche de guidage orthopédique, de manière à rapprocher la fraise vis-à-vis d'un os à fraiser non pas frontalement à cet os, mais latéralement à celui-ci. De cette façon, dans le cas d'une fraiseuse glénoïdienne, la fraise peut être glissée ou insérée entre la tête de l'humérus et l'omoplate du patient, sans avoir à distraire trop fortement l'épaule du patient, ni à écarter les tissus mous entourant la glène sur toute la périphérie de cette dernière, jusqu'à centrer la fraise avec la broche de guidage. Bien entendu, pour arriver jusqu'à ce centrage, la broche de guidage doit traverser radialement la paroi du moyeu, ce qui est rendue possible selon l'invention en engageant la broche de guidage à travers une fente du moyeu, dimensionnée à cet effet. Avec la fraiseuse selon l'invention, la fraise est mise en place sur l'os, en particulier sur la glène, de manière simplifiée et peu invasive, tout en pouvant être ensuite actionnée pour fraiser l'os en étant appliquée avec précision à l'aide de la broche de guidage. En pratique, l'actionnement de la fraise, c'est-à-dire son entraînement en rotation ou en oscillation sur elle-même autour de l'axe de son moyeu, peut être soit motorisé, soit manuel : dans les deux cas, le moyeu de la fraise peut avantageusement être couplé à un moyen d'entraînement ad hoc, enfilé axialement autour de la broche de guidage.

Bien entendu, le dégagement de la fraise selon l'invention s'effectue tout aussi aisément, en la déplaçant latéralement à la glène de manière à désengager son moyeu de la broche de guidage, via la fente du moyeu.

Des caractéristiques additionnelles avantageuses de l'ensemble conforme à l'invention sont spécifiées aux revendications dépendantes 2 à 10.

L'invention sera mieux comprise à la lecture de la description qui va suivre, donnée uniquement à titre d'exemple et faite en se référant aux dessins sur lesquels :
- la figure 1 est une vue en perspective d'un premier mode de réalisation d'une fraise appartenant à un ensemble conforme à l'invention ;
- la figure 2 est une vue en élévation selon la flèche II de la figure 1 ;
- la figure 3 est une vue en perspective de la fraise des figures 1 et 2, associée à une omoplate dont la glène est à fraiser, la figure 3 illustrant ainsi une première étape d'utilisation de la fraise ;
- les figures 4 à 6 sont des vues analogues à la figure 3, illustrant respectivement des opérations successives de mise en oeuvre de la fraise sur la glène de l'omoplate ;
- les figures 7 et 8 sont des vues analogues à la figure 2, illustrant respectivement deux variantes du premier mode de réalisation de la fraise, conformes à l'invention ;
- la figure 9 est une vue en perspective d'un second mode de réalisation d'une fraise appartenant à un ensemble conforme à l'invention ;
- la figure 10 est une vue en élévation selon la flèche X de la figure 9 ;
- la figure 11 est une vue en perspective d'un troisième mode de réalisation d'une fraise appartenant à un ensemble conforme à l'invention ; et
- la figure 12 est une vue en élévation selon la flèche XII de la figure 11.

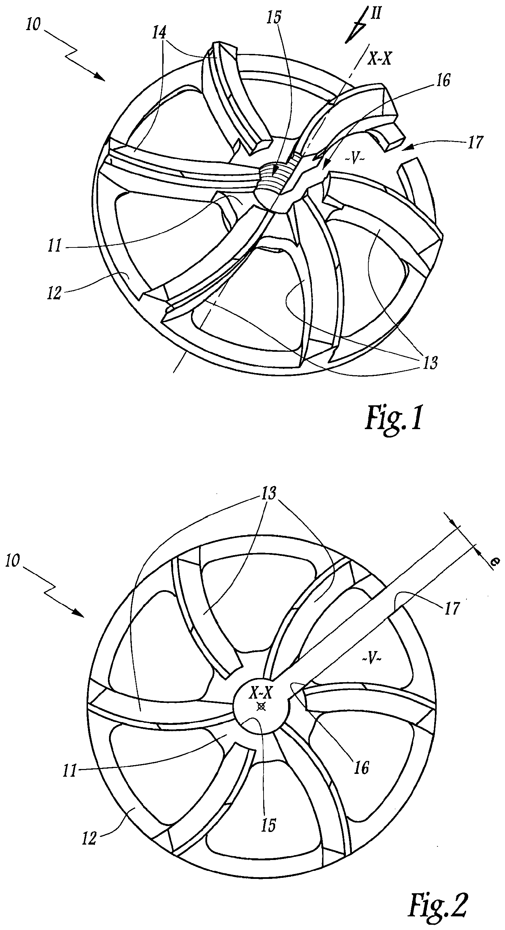

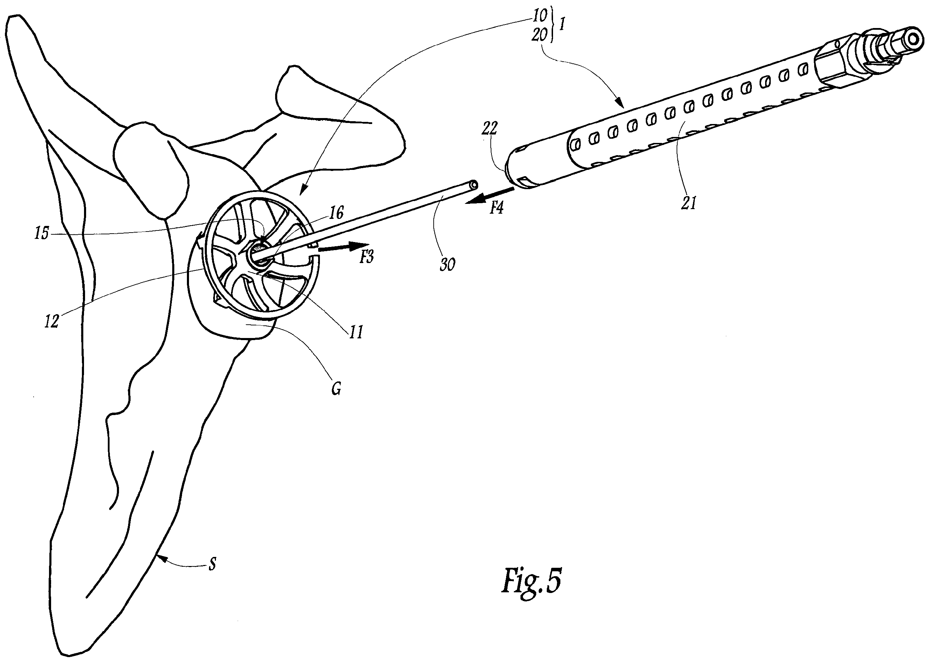

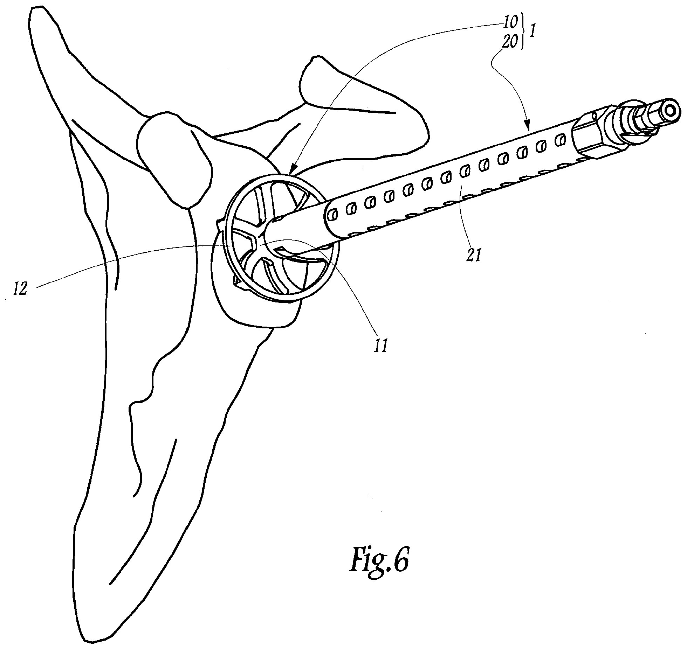

Sur les figures 1 à 6 est représentée une fraiseuse 1 qui comporte une fraise 10, représentée seule sur les figures 1 et 2, et un moyen 20 d'entraînement de la fraise 10, montré sur les figures 5 et 6. Le moyen d'entraînement 20 sera détaillé plus loin, en regard des figures 5 et 6.

Comme bien visible sur les figures 1 à 3, la fraise 10 présente une forme globalement discoïdale à base circulaire, centrée sur un axe X-X. Plus précisément, la fraise 10 inclut deux parties annulaires concentriques, à savoir un moyeu interne 11 et une couronne externe 12, tous les deux centrés sur l'axe X-X. Le moyeu 11 et la couronne 12 sont reliés rigidement l'un à l'autre par six pattes 13 individuellement identiques, qui s'étendent chacune en longueur depuis le moyeu 11 suivant une direction sensiblement radiale à l'axe X-X et qui sont réparties de manière sensiblement uniforme autour du moyeu. Dans l'exemple de réalisation représenté sur les figures, ces pattes 13 ne sont pas rigoureusement rectilignes, mais présentent, suivant leur longueur, une légère courbure. En pratique, les pattes 13 peuvent présenter des profils longitudinaux de formes variées, non limitatives de la présente invention.

Sur leur face distale, c'est-à-dire celle tournée vers le lecteur regardant les figures 1 et 2, les pattes 13 sont chacune pourvues d'une lame 14 qui court sur toute la longueur de la patte. Les lames 14 sont conçues pour attaquer de la matière osseuse et ainsi la fraiser lorsque la fraise 10 est entraînée en rotation sur elle-même autour de son axe X-X. A cet effet, chaque lame 14 est, par exemple, munie d'une arrête coupante s'étendant sur le chant d'extrémité distale de la patte correspondante 13. Avantageusement, les lames 14 ne s'étendent pas rigoureusement dans un plan perpendiculaire à l'axe X-X, mais appartiennent à une enveloppe sphérique qui est bombée en direction du côté distal de la fraise 10 et dont l'axe X-X constitue un axe de révolution : de cette façon, lors de l'entraînement de la fraise 10 en rotation sur elle-même autour de l'axe X-X, les lames 14 sont à même de creuser dans de la matière osseuse une cavité sensiblement sphérique, à révolution centrée sur l'axe X-X.

Bien entendu, en pratique, d'autres formes de réalisation que les lames 14 sont envisageables en tant que reliefs d'attaque osseuse, comme par exemple des picots, des dents, des pointes, etc. D'ailleurs, à titre de variante non représentée, de tels reliefs d'attaque osseuse peuvent être prévus sur tout ou partie de la face distale de la couronne 12, ainsi que sur tout ou partie de la face distale du moyeu 11.

Autrement dit, de manière plus générale, la couronne 12 et les pattes 13 forment, au moins en partie, un corps de fraisage, qui est agencé co-axialement et transversalement au moyeu 11 et qui peut prendre diverses formes de réalisation, non limitatives de la présente invention.

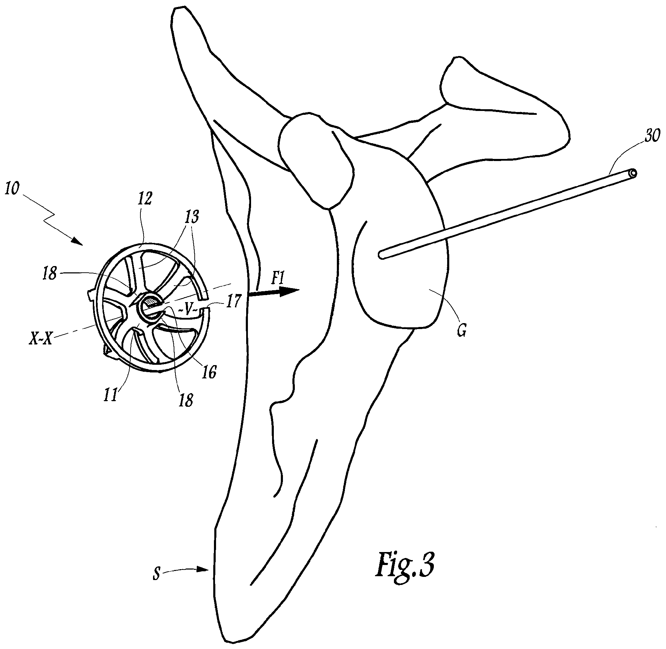

Eu égard à sa forme annulaire, le moyeu 11 délimite intérieurement un alésage 15 qui est centré sur l'axe X-X et qui traverse axialement le moyeu 11 de part en part, en débouchant ainsi sur la face distale du moyeu, comme bien visible sur les figures 1 et 2, et sur la face proximale du moyen, comme bien visible sur la figure 3. Pour des raisons qui apparaîtront plus loin, l'alésage 15 est intérieurement taraudé.

Comme bien visible sur les figures 1 à 3, la paroi annulaire du moyeu 11, qui délimite l'alésage 15, est fendue sur toute sa dimension axiale, en une portion seulement de sa périphérie. Autrement dit, le moyen 11 délimite une fente transversale 16 qui, suivant la direction de l'axe X-X, relie les faces distale et proximale du moyen et qui, suivant une direction radiale à l'axe X-X, relie l'alésage 15 et la face périphérique extérieure du moyeu 11. Dans un plan perpendiculaire à l'axe X-X, les bords opposés de la fente 16 sont distants l'un de l'autre, avec un écartement noté e sur la figure 2. Dans l'exemple de réalisation des figures 1 à 3, les bords de la fente 16 sont globalement plans, en s'étendant de manière sensiblement parallèle à un plan diamétral de la fraise 10 passant par l'axe X-X. A titre de variante non représentée, les bords précités de la fente peuvent présenter une certaine courbure, en particulier avec un profil incurvé dans un plan perpendiculaire à l'axe X-X, du moment que ces bords maintiennent entre eux l'écartement e dans un plan perpendiculaire à l'axe X-X.

Dans la forme de réalisation des figures 1 à 3, la fente 16 est prévue dans une portion du moyeu 11, qui est située, suivant la périphérie du moyeu, entre deux pattes 13 successives, plus précisément entre les extrémités de ces deux pattes 13 successives, tournées vers l'axe X-X. De la sorte, radialement à l'opposé de son débouché dans l'alésage 15, la fente 16 débouche dans un volume libre V du corps de fraisage de la fraise 10, qui est délimité par les deux pattes successives 13 précitées et par la portion périphérique de la couronne 12, reliant les extrémités des pattes successives 13 précitées, opposées à l'axe X-X. De plus, dans la portion précitée de la couronne 12 est délimité un passage transversal 17, qui traverse radialement de part en part la couronne 12, en reliant ainsi le volume libre V et la face périphérique extérieure de la couronne 12. Ce passage 17 s'apparente donc à une fente traversante, réalisée radialement à travers la couronne 12. Pour des raisons précisées plus loin, ce passage 17 est avantageusement disposé de manière sensiblement aligné avec la fente 16 suivant une direction radiale à l'axe X-X, comme bien visible sur la figure 2.

Les bords opposés du passage 17 sont, dans un plan perpendiculaire à l'axe X-X, distants l'un de l'autre, avec un écartement relatif sensiblement égal à l'écartement e entre les bords de la fente 16.

Un exemple d'utilisation de la fraise 10 va maintenant être présenté en regard des figures 3 à 6, dans le cadre d'une intervention chirurgicale visant à préparer la glène G d'une omoplate S en vue d'y implanter un composant glénoïdien d'une prothèse d'épaule.

Initialement, comme représenté sur la figure 3, on considère que, préalablement à l'utilisation proprement dite de la fraise 10, une broche de guidage orthopédique 30 est mise en place dans la glène G. Cette broche de guidage 30 consiste en une tige rigide de petite section transversale, qui est, sur une partie terminale distale, enfoncée dans la matière osseuse constituant la glène G. En pratique, le point d'enfoncement de la broche 30 dans la glène G, ainsi que la direction de cet enfoncement vis-à-vis de la glène sont imposés par le chirurgien, en ayant été préalablement déterminés de manière précise, dans le sens où la broche de guidage 30 matérialise un axe d'implantation préférentiel du composant prothétique glénoïdien précité. En effet, à l'aide de la broche de guidage 30, le chirurgien va, au cours de l'intervention chirurgicale visant à implanter le composant glénoïdien, utiliser plusieurs ancillaires chirurgicaux dont l'application sur la glène G va être guidée par la broche 30. La mise en place de la broche de guidage 30 étant une opération bien connue en chirurgie orthopédique de l'épaule, elle se sera pas décrite ici plus en détail.

Comme représenté sur la figure 3, le chirurgien approche la fraise 10 de la glène G à fraiser, non pas en enfilant axialement l'alésage 15 autour de la broche de guidage 30, mais en déplaçant la fraise 10 latéralement à la broche de guidage 30, globalement dans un plan perpendiculaire à cette broche. Cette approche latérale de la broche de guidage 30 par la fraise 10 est indiquée par une flèche F1 sur la figure 3 : cette approche latérale permet de glisser la fraise 10 entre l'omoplate S et la tête de l'humérus, non représentée, associée à cette omoplate, alors que la face distale de la fraise est tournée vers l'omoplate. De la sorte, il n'est pas nécessaire pour le chirurgien de distraire trop fortement l'articulation de l'épaule, limitant ainsi le trauma de cette articulation.

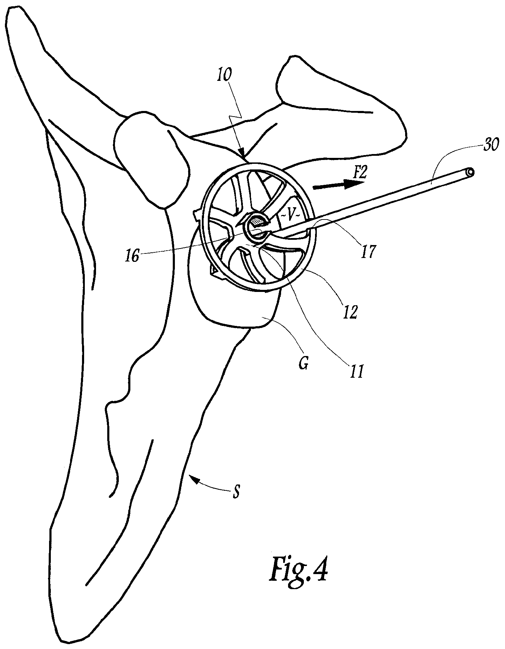

Comme bien visible par comparaison des figures 3 et 4, l'approche latérale de la broche de guidage 30 par la fraise 10 est poursuivie jusqu'à ce que la couronne 12 de la fraise 10 vienne à proximité immédiate de la broche de guidage 30, plus précisément de la partie courante de cette broche de guidage 30, émergeant de la glène G. Le déplacement de la fraise 10 est alors poursuivi, comme indiqué par la flèche F2 à la figure 4, de manière à engager transversalement la broche de guidage 30 à travers le passage 17, globalement suivant une direction radiale à l'axe X-X.

Bien entendu, pour que la broche de guidage 30 puisse ainsi passer à travers la couronne 12 via le passage 17, l'écartement entre les bords de ce passage est prévu au moins égal, voire légèrement supérieur au diamètre de la broche de guidage 30. Après avoir franchi le passage 17, la broche de guidage 30 se trouve positionnée à l'intérieur de la couronne 12, en s'étendant axialement au travers du volume libre V.

Toujours en poursuivant l'approche latérale de la broche de guidage 30 par la fraise 10, le chirurgien rapproche le moyeu 11 de la partie courante de cette broche de guidage 30, jusqu'à engager cette partie courante de la broche de guidage à travers la fente 16, comme indiqué par la flèche F3 sur la figure 5. Suivant les mêmes considérations qui précèdent en ce qui concerne le passage 17, on comprend que l'écartement e entre les bords de la fente 16 est suffisant, comparativement au diamètre de la broche 30, pour permettre le passage de cette broche de guidage à travers la paroi du moyeu 11, via la fente 16, jusqu'à ce que la broche de guidage 30 se retrouve à l'intérieur de l'alésage 15, notamment dans une configuration coaxiale à cet alésage, comme représenté à la figure 5. Autrement dit, dans cette configuration, l'axe X-X de la fraise 10 se trouve confondu avec l'axe central de la broche de guidage 30. Ainsi, on notera que, moyennant l'approche latérale qui vient d'être décrite en regard des figures 3 à 5, la fraise 10 a été déplacée jusqu'à la rendre coaxiale à la broche de guidage 30, sans avoir eu besoin d'engager axialement cette fraise 10 depuis l'extrémité proximale de la broche de guidage 30. Ce mouvement d'approche latérale permet au chirurgien de limiter l'étendue des incisions chirurgicales dans les tissus mous entourant la glène G, dans le sens où ces tissus mous n'ont à être incisés et écartés que sur environ une demie périphérie de la glène G.

L'utilisation de la fraise 10 est poursuivie en rapportant le moyen d'entraînement 20. Comme représenté sur les figures 5 et 6, ce moyen d'entraînement 20 se présente sous la forme d'un manche tubulaire 21 qui est conçu pour être enfilé autour de la broche de guidage 30, en étant engagé axialement autour de cette broche de guidage depuis l'extrémité proximale de cette dernière, comme bien visible par comparaison des figures 5 et 6. Le manche tubulaire 21 est coulissé autour de la broche de guidage 30, en direction de la fraise 10, comme indiqué par la flèche F4 sur la figure 5. Ce mouvement de coulissement est poursuivi jusqu'à ce que l'extrémité distale du manche 21 se retrouve, le long de la broche de guidage 30, au niveau de la fraise 10. L'extrémité 22 du manche 21 est alors couplée mécaniquement au moyeu 11 : dans l'exemple de réalisation considéré sur les figures, ce couplage est réalisé par vissage de l'extrémité 21, filetée à cet effet, dans le taraudage de l'alésage 15. Bien entendu, d'autres formes de couplage mécanique entre le moyen d'entraînement 20 et le moyeu 11 peuvent être envisagées dans le cadre de la présente invention.

Avantageusement, lors des opérations de couplage, le chirurgien manipule la fraise 10 en l'agrippant par l'extrémité proximale du moyeu 11 : dans l'exemple de réalisation considéré sur les figures, l'extrémité proximale du moyen 11 délimite à cet effet deux méplats 18 diamétralement opposés, bien visibles sur la figure 5, qui permettent au chirurgien d'immobiliser fermement la fraise 10 en rotation autour de l'axe X-X, pour faciliter le vissage de l'extrémité 22 du manche 21 dans l'alésage 15. D'ailleurs, ces méplats 18 peuvent être utilisés pas le chirurgien pour, le cas échéant à eux seuls, manipuler la fraise 10 lors de toutes les étapes de sa mise en place, illustrées par les figures 3 à 6.

Une fois que le moyen d'entraînement 20 est solidarisé à la fraise 10, comme représenté sur la figure 6, ce moyen d'entraînement 20 est mis en rotation sur lui-même autour de son axe longitudinal central, par exemple en reliant son extrémité proximale à un moteur d'entraînement ad hoc. Le mouvement de rotation imposé au manche 21 est transmis à la fraise 10 dont les lames 14 attaquent alors la matière osseuse constituant la glène G, afin de la fraiser, comme évoqué plus haut. Bien entendu, plutôt que d'être motorisé, l'entraînement du moyen 20 peut être manuel.

Le fraisage de la glène G par la fraise 10 est ainsi guidé par la broche de guidage 30, dans la mesure où cette broche de guidage matérialise l'axe d'application de la fraise 10 sur la glène G. Une fois le fraisage de la glène G terminé, le moyen d'entraînement 20 est, après désaccouplement entre le manche 21 et le moyeu 11, dégagé par coulissement le long de la broche de guidage 30 en direction de l'extrémité proximale de cette broche. Puis, le chirurgien dégage la fraise 10 latéralement à la broche de guidage 30, en faisant passer la partie courante de cette broche de guidage successivement à travers la fente 16 et le passage 17, suivant un mouvement latéral opposé à celui décrit en regard des figures 3 à 5.

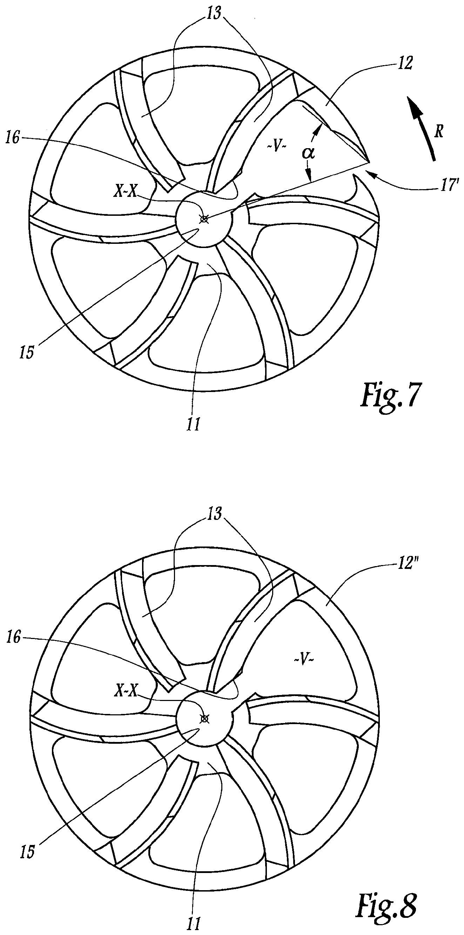

Sur la figure 7 est représentée une variante de la fraise 10, dont les éléments communs avec la forme de réalisation des figures 1 à 6 portent les mêmes références. Cette variante de la figure 7 ne se distingue de la forme de réalisation des figures 1 à 6 que par le passage transversal prévu à travers la couronne 12, ce passage étant référencé 17' à la figure 7. Plus précisément, le passage 17' se distingue du passage 17 par le fait que, contrairement au passage 17, ses bords ne s'étendent pas globalement dans des plans parallèles à un plan diamétral de la fraise 10 passant par l'axe X-X, mais sont, dans un plan perpendiculaire à cet axe X-X, fortement inclinés par rapport à une direction radiale à cet axe X-X. Ainsi, dans un plan perpendiculaire à l'axe X-X, la droite reliant les extrémités intérieure et extérieure de chacun des bords du passage 17' forment, avec une direction radiale à l'axe X-X, un angle a non nul, dont la valeur est prévue supérieure à 30°, voire 45°. De la sorte, le passage 17' ne s'étend pas, entre ses débouchés sur l'extérieur de la couronne 12 et sur le volume libre V, rigoureusement suivant une direction radiale à l'axe X-X, mais selon une trajectoire inclinée à cette direction radiale : par comparaison au passage 17, cela fait que le débouché sur l'extérieur du passage 17' a moins tendance à interférer avec, typiquement à agripper, des éléments entourant extérieurement la couronne 12 lorsque la fraise 10 est entraînée en rotation dans le sens indiqué par la flèche R sur la figure 7, c'est-à-dire en sens opposé à l'inclinaison α du passage 17' par rapport à la direction radiale à l'axe X-X. En effet, le bord arrière, suivant le sens rotatif R, du passage 17' forme, avec la face périphérique extérieure de la couronne 12, un angle obtus, supérieur à 135°, qui tend à repousser vers l'extérieur du passage 17' des éléments qui auraient tendance à y rentrer lors de la rotation de la fraise 10. De tels éléments sont en particulier les tissus mous entourant la glène G : autrement dit, avec la forme de réalisation de la figure 7, les risques que le passage 17' agrippent ou accrochent les tissus mous entourant la glène lors de la mise en rotation de la fraise 10 suivant le sens R sont limités, comparativement à la forme de réalisation de la fraise 10 des figures 1 à 6. Avantageusement, plutôt que d'être rigoureusement plans, les bords du passage 17' présentent, dans un plan de coupe perpendiculaire à l'axe X-X, un profil incurvé, bombé en direction opposée à l'axe X-X. Cela renforce l'effet de non accrochage des tissus mous potentiellement présents autour de la couronne 12 lorsque la fraise 10 est entraînée en rotation selon le sens de rotation R.

On notera que l'inclinaison α du passage 17' par rapport à la direction radiale à l'axe X-X fait que son débouché sur l'extérieur est décalé, suivant une direction périphérique de la couronne 12, par rapport à son débouché sur le volume libre V. Par conséquent, lors de la mise en place de la fraise 10 sur la glène G à fraiser, les manipulations de cette fraise doivent en tenir compte, à la différence de la mise en place de la forme de réalisation des figures 1 à 6, avec laquelle l'engagement de la broche de guidage 30 à travers le passage 17 puis la fente 16 consiste plus simplement en un déplacement sensiblement rectiligne de la fraise 10 par rapport à la broche de guidage, suivant une direction sensiblement radiale à l'axe X-X.

Sur la figure 8 est représentée une autre variante de la fraise 10 des figures 1 à 6, dont les composants communs à la forme de réalisation des figures 1 à 6 portent les mêmes références numériques. La variante de la figure 8 se distingue de la fraise des figures 1 à 6 par l'absence du passage 17 : autrement dit, à la différence de la couronne 12, la couronne 12" de la fraise 10 de la figure 8 est ininterrompue suivant toute sa périphérie. L'absence du passage 17 facilite la fabrication de la fraise 10, mais elle induit une utilisation légèrement différente de celle de la forme de réalisation des figures 1 à 6. En effet, dans la mesure où la couronne 12" n'est pas fendue, il n'est pas possible d'amener la broche de guidage 30 en travers du volume libre V moyennant une approche latérale de cette broche de guidage. Aussi, à la différence de ce qui a été décrit plus haut en regard des figures 3 et 4, le chirurgien engage axialement la broche de guidage 30 dans le volume libre V, en engageant axialement l'extrémité proximale de cette broche de guidage entre les deux pattes successives 13 délimitant ce volume libre V. Puis, le chirurgien fait coulisser la fraise 10 le long de la broche de guidage 10 alors que la broche de guidage 30 s'étend en travers du volume libre V, de préférence alors que la broche de guidage est plaquée latéralement contre la face périphérique intérieure de la couronne 12" : de cette façon, le chirurgien amène la fraise 10 jusqu'à proximité de la glène G tout en maintenant la fraise 10 excentrée par rapport à la broche de guidage 30, de préférence de manière la plus excentrée possible. Ce faisant, le chirurgien peut jouer sur l'inclinaison de la fraise 10 pour contourner la tête de l'humérus, ainsi que d'autres organes du patient, tout en limitant l'étendue des incisions et des écartement des tissus mous entourant la glène. Une fois que la fraise 10 est coulissée jusqu'au niveau de la partie courante de la broche de guidage 30 émergeant de la glène 10, le chirurgien approche le moyeu 11 de la broche de guidage 30 latéralement à cette dernière, exactement de la même façon que décrit précédemment en regard de la figure 5, de manière à engager cette partie courante de la broche de guidage 30 à travers la fente 16, jusqu'à rendre l'alésage 15 coaxial avec la broche de guidage.

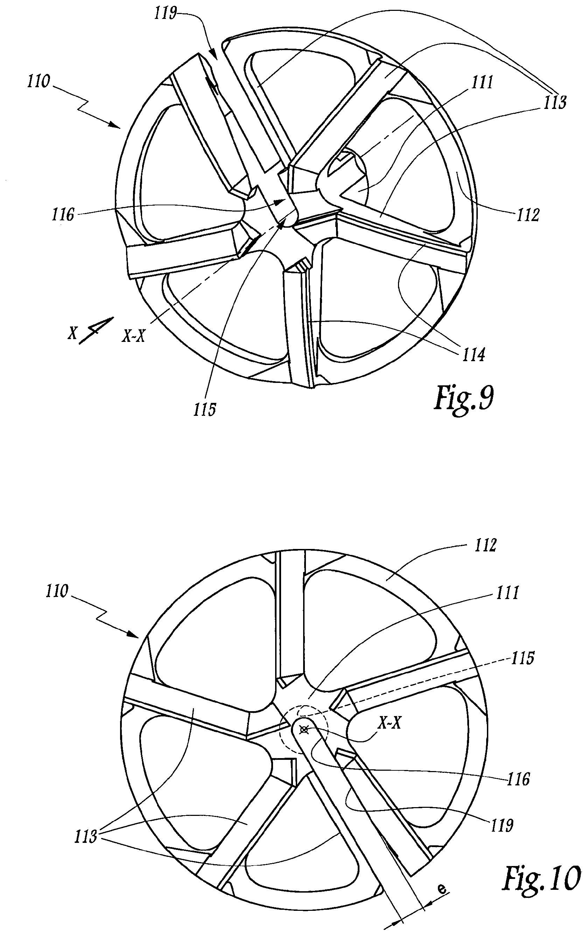

Sur les figures 9 et 10 est représentée une fraise 110 constituant un mode de réalisation alternatif de la fraise 10. Cette fraise 110 est notamment conçue pour être utilisée avec le moyen d'entraînement 20 décrit plus haut, au sein d'une fraiseuse fonctionnellement similaire à la fraiseuse 1.

De manière similaire à la fraise 10, la fraise 110 comporte un moyeu annulaire intérieur 111, qui est centré sur l'axe X-X et qui délimite intérieurement un alésage traversant 115, et un corps de fraisage qui s'étend transversalement et autour du moyeu 111. Ce corps de fraisage inclut une couronne externe 112 et cinq pattes 113, qui relient le moyeu 111 et la couronne 112 suivant une direction sensiblement radiale à l'axe X-X et qui sont répartis autour de cet axe suivant la périphérie du moyeu 111. De manière similaire aux pattes 13 de la fraise 10, les pattes 113 de la fraise 110 sont pourvues, sur leur face distale, de lames de découpe 114.

Les pattes 113 se distinguent des pattes 13 par le fait que, dans un plan de coupe perpendiculaire à l'axe X-X, elles présentent un profil sensiblement rectiligne. De surcroît, à la différence des pattes 13 qui sont toutes individuellement identiques, les pattes 113 sont réparties en un groupe de quatre pattes 113 identiques, tandis que la patte 113 restante se différencie des autres pattes 113 par la présence d'une ouverture 119 qui, d'une part, traverse de part en part la patte 113 suivant la direction de l'axe X-X, en reliant ainsi les faces distale et proximale de cette patte, et, d'autre part, court sur toute la dimension radiale de la branche 113, en reliant ainsi les extrémités intérieure et extérieure de cette patte. Plus précisément, à l'extrémité extérieure de la patte 113 précitée, l'ouverture 119 débouche sur la face périphérique extérieure de la couronne 112, tandis que, à l'extrémité intérieure de cette patte, l'ouverture 119 est prolongée par une fente 116, fonctionnellement similaire à la fente 16, délimitée à travers la paroi annulaire du moyeu 111. Comme bien visible sur la figure 10, dans un plan perpendiculaire à l'axe X-X, l'ouverture 119 est inscrite dans le prolongement rectiligne de la fente 116, ses bords opposés étant distants l'un de l'autre avec un écartement sensiblement égal à l'écartement e entre les bords de la fente 116.

La fraise 110 s'utilise sensiblement de la même façon que la fraise 10 : avant de coupler le moyeu 111 au moyen d'entraînement 20 coulissé le long de la broche de guidage 30, la fraise 110 est approchée de cette broche de guidage 30 latéralement à la partie courante de celle-ci, de manière à engager radialement cette partie courante de la broche de guidage 30 à travers, successivement, l'ouverture 119 et la fente 116, jusqu'à atteindre l'intérieur de l'alésage 115.

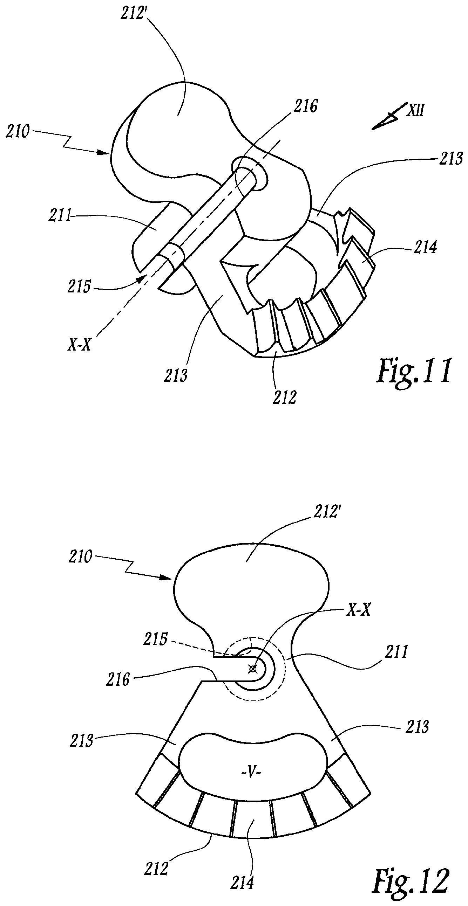

Sur les figures 11 et 12 est représentée une fraise 210 consistant en une alternative de réalisation des fraises 10 et 110. En pratique, de manière similaire aux fraises 10 et 110, la fraise 210 est prévue pour être couplée au moyen d'entraînement 20 au sein d'une fraiseuse similaire à la fraiseuse 1.

La fraise 210 comporte un moyeu annulaire 211, centré sur un axe X-X et fonctionnellement similaire au moyeu 11 ou 111 des fraises 10 ou 110.

La fraise 210 se distingue des fraises 10 et 110 par la forme globale de son corps de fraisage : en effet, ce corps ne s'étend que sur seulement une portion périphérique du moyen 211 et consiste en une portion de couronne 212 dont chacune des extrémités périphériques est reliée au moyen 111 par une patte radiale 213. Sur sa face distale, la portion de couronne 212 est pourvue d'une denture 214 d'attaque de la matière osseuse glénoïdienne. Ainsi, le corps de fraisage de la fraise 210 présente un encombrement global nettement moindre que celui du corps de fraisage des fraises 10 et 110. On comprend ainsi que la fraise 210 est préférentiellement utilisée pour réaliser des préparations osseuses de moindre ampleur.

Pour des raisons d'équilibrage, la fraise 210 comporte avantageusement une masselotte 212', qui s'étend transversalement depuis le moyeu 211 et qui est agencée de manière diamétralement opposée à la portion de couronne 212.

De manière similaire au moyeu 11 ou 111 des fraises 10 et 110, le moyeu 211 de la fraise 210 délimite, à la fois, un alésage intérieur 215, centré sur l'axe X-X, et une fente latérale 216, qui relie, suivant une direction radiale à l'axe X-X, l'intérieur de l'alésage 215 et l'extérieur du moyeu 211. Comme bien visible sur la figure 12, cette fente 216 est située, suivant la périphérie du moyeu 211, en dehors du corps de fraisage, c'est-à-dire en dehors de la portion délimitée par les deux pattes 213, dans laquelle s'étend la portion de couronne 212. Plus précisément, dans l'exemple de réalisation considéré sur les figures 11 et 12, la fente 216 s'étend selon une direction radiale à l'axe X-X, qui est agencée sensiblement à 90° de la direction diamétrale selon laquelle sont opposées la portion de couronne 212 et la masselotte 212'. A titre de variante non représentée, la fente 216 peut être située dans la même portion que la portion de couronne 212 et elle débouche alors dans le volume libre V entre les deux pattes 213.

L'utilisation de la fraise 210 est similaire à celle décrite plus haut pour les fraises 10 et 110, dans le sens où la mise en place de la fraise 210 sur une glène à fraiser, préalablement pourvue de la broche de guidage 30, consiste à rapprocher la fraise 210 de cette broche de guidage latéralement à la partie courante de cette broche de guidage 30, de manière à engager radialement cette partie courante de la broche de guidage à travers la fente 216, jusqu'à atteindre l'intérieur de l'alésage 215.

Divers aménagements et variantes à la fraiseuse décrite ci-dessus, en particulier aux fraises 10, 110 et 210, sont par ailleurs envisageables. A titre d'exemples :
- à titre de variantes non représentées pour les fraises 10 et 110, leur couronne 12 ou 12" et 112 peuvent être supprimées en une ou plusieurs portions suivant leur périphérie, voire sur toute leur périphérie, en particulier pour des fraises de petite taille ;
- à titre de variante non représentée pour la fraise 210, la masselotte 212' peut être remplacée par une portion de couronne dentée, symétrique de la portion de couronne 212 par rapport à l'axe X-X ;
- plutôt que d'être à base circulaire, le corps de fraisage des fraises 10, 110 et 210 peut être centré sur l'axe X-X en présentant une base ovale ou ovoïde ou multilobée ;
- comme évoqué plus haut, la géométrie de la cavité creusée par les fraises 10, 110 et 210 dans de la matière osseuse n'est pas limitative de l'invention; ainsi, moyennant des aménagements ad hoc de la face distale de leur corps de fraisage, ces fraises peuvent réaliser aussi bien une activité sphérique concave comme décrit plus haut, qu'un relief sphérique convexe, une résection plane, une cavité à région centrale sphérique et à bord périphérique plat, etc. ; et/ou
- l'invention peut être appliquée à des fraiseuses orthopédiques destinées à être utilisées sur d'autres os que la glène d'une omoplate ; ainsi, l'invention est par exemple applicable au fraisage de l'humérus ou des os de la main ou du pied.

## Revendications

1. Ensemble de préparation osseuse, en particulier de préparation glénoïdienne, comportant :
- une broche de guidage orthopédique (30) ayant une partie terminale distale qui est conçue pour être enfoncée dans une matière osseuse, et
- une fraiseuse orthopédique (1) comportant une fraise (10 ; 110 ; 210) qui inclut un moyeu (11 ; 111 ; 211) et un corps de fraisage (12, 13 ; 112, 113 ; 212, 213) pourvu de reliefs d'attaque osseuse (14 ; 114 ; 214) ;
lequel moyeu est muni d'un alésage axial (15 ; 115 ; 215) de réception de la broche (30), cet alésage débouchant sur les faces axialement opposées, respectivement proximale et distale, du moyeu ;
lequel moyeu délimite une fente (16 ; 116 ; 216) de passage latéral pour la broche de guidage (30), cette fente débouchant de manière sensiblement radiale dans l'alésage et reliant les faces proximale et distale du moyeu ; et
lequel corps de fraisage s'étend transversalement au moyeu, en entourant extérieurement la périphérie du moyeu au moins en partie, tout en laissant ouvert le débouché de la fente, radialement opposé à l'alésage,
**caractérisé en ce que** le corps de fraisage (12,13 ; 112, 113 ; 212, 213) comporte à la fois une couronne (12 ; 12"; 112) ou une portion de couronne (212), radialement distante du moyeu, et des pattes (13 ; 113 ; 213), qui relient le moyeu et la couronne ou portion de couronne suivant une direction sensiblement radiale et qui sont réparties autour du moyeu (11 ; 111 ; 211),
et **en ce que** le moyeu (11 ; 111 ; 211) est en outre prévu pour, alors que la partie terminale distale de la broche (30) est en place dans une matière osseuse, être approché latéralement de la broche au niveau d'une extrémité, émergeant de la matière osseuse, de cette partie terminale distale de manière que cette extrémité de la partie terminale distale de la broche soit engagée radialement à travers la fente (16 ; 116 ; 216) jusqu'à atteindre l'intérieur de l'alésage (15; 115 ; 215).

2. Ensemble suivant la revendication 1, **caractérisé en ce que** le corps de fraisage (12, 13 ; 112, 113) s'étend tout autour du moyeu (11 ; 111).

3. Ensemble suivant la revendication 2, **caractérisée**n **ce que** la fente (16) débouche, radialement à l'opposé de l'alésage (15), dans un volume libre (V) délimité entre deux pattes (13) qui se succèdent autour du moyeu (11).

4. Ensemble suivant la revendication 3, **caractériséen ce que** la couronne (12) délimite un passage latéral (17 ; 17') pour la broche de guidage (30), lequel passage relie ledit volume libre (V) et la face périphérique extérieure de la couronne.

5. Ensemblesuivant la revendication 4, **caractérisée**n **ce que** le passage (17) et la fente (16) sont situés de manière sensiblement alignée suivant une direction radiale à l'axe (X-X) du moyeu (11).

6. Ensemble suivant la revendication 4, **caractériséen ce que** les bords du passage (17') sont inclinés d'au moins 30° par rapport à une direction radiale à l'axe (X-X) du moyeu.

7. Ensemble suivant la revendication 3, **caractérisée**n **ce que** la couronne (12") est ininterrompue suivant toute sa périphérie.

8. Ensemble suivant la revendication 1, **caractérisée**n **ce que** l'une des pattes (113) délimite une ouverture (119) de réception latérale pour la broche de guidage (30), laquelle ouverture relie la face périphérique extérieure de la couronne (112) et la fente (116).

9. Ensemble suivant la revendication 1, **caractérisé en ce que** le corps de fraisage (212, 213) s'étend sur seulement une portion périphérique du moyeu (211), en dehors de laquelle la fente (216) est située.

10. Ensemble suivant l'une quelconque des revendications précédentes, **caractérisé en ce que** la fraiseuse (1) comporte en outre un moyen (20) d'entraînement de la fraise (10 ; 110 ; 210), qui est adapté pour être simultanément engagé autour de la broche de guidage (30) et couplé au moyeu (11 ; 111 ; 211) à des fins d'entraînement de la fraise autour de l'axe (X-X) de son moyeu.

## Patentansprüche

1. Anordnung zur Knochenpräparation, insbesondere Gelenkpräparation, umfassend:
- einen orthopädischen Führungsdorn (30), der einen distalen Endabschnitt aufweist, der ausgebildet ist, in ein Knochenmaterial eingetrieben zu werden, und
- eine orthopädische Fräsvorrichtung (1), die eine eine Nabe (11; 111; 211) und einen Fräskörper (12, 13; 112, 113; 212, 213) einschließende Fräse (10; 110; 210) aufweist, wobei der Fräskörper mit Knochenangriffserhöhungen (14; 114; 214) versehen ist;
wobei die Nabe mit einer axialen Bohrung (15; 115; 215) zur Aufnahme des Dorns (30) ausgerüstet ist und diese Bohrung an den axial entgegengesetzten Flächen der Nabe, jeweils der proximalen und distalen, mündet;
wobei die Nabe einen Spalt (16; 116; 216) für den lateralen Durchgang des Führungsdorns (30) begrenzt, wobei dieser Spalt im Wesentlichen radial in die Bohrung mündet und die proximale und distale Fläche der Nabe verbindet; und
wobei der Fräskörper sich quer zur Nabe erstreckt, indem er außen den Umfang der Nabe mindestens teilweise umgibt, dabei die Mündung des Spaltes radial gegenüberliegend zur Bohrung offen lassend,
**dadurch gekennzeichnet, dass** der Fräskörper (12, 13; 112, 113; 212, 213) sowohl einen Kranz (12; 12"; 112) oder einen Teil eines Kranzes (212), radial beabstandet zur Nabe, als auch Speichen (13; 113; 213) aufweist, die die Nabe und den Kranz oder einen Kranzbereich gemäß einer im Wesentlichen radialen Richtung verbinden und die um die Nabe (11; 111; 211) herum verteilt sind,
und dass die Nabe (11; 111; 211) außerdem vorgesehen ist, wenn der distale Endabschnitt des Dorns (30) in einem Knochenmaterial in Stellung ist, seitlich an den Dorn an ein aus dem Knochenmaterial auftauchenden Ende dieses distalen Endabschnitts angenähert zu werden, derart, dass dieses Ende des distalen Endabschnitts des Dorns radial durch den Spalt (16; 116; 216) eingreift, bis das Innere der Bohrung (15; 115; 215) erreicht ist.

2. Anordnung nach Anspruch 1, **dadurch gekennzeichnet, dass** der Fräskörper (12, 13; 112, 113) sich ganz um die Nabe (11; 111) herum erstreckt.

3. Anordnung nach Anspruch 2, **dadurch gekennzeichnet, dass** der Spalt (16) radial gegenüberliegend zur Bohrung (15) in einen freien Raum (V) mündet, der zwischen zwei um die Nabe (11) herum aufeinanderfolgenden Speichen (13) begrenzt ist.

4. Anordnung nach Anspruch 3, **dadurch gekennzeichnet, dass** der Kranz (12) einen seitlichen Durchgang (17; 17') für den Führungsdorn (30) begrenzt, wobei der Durchgang den freien Raum (V) und die äußere Umfangsfläche des Kranzes verbindet.

5. Anordnung nach Anspruch 4, **dadurch gekennzeichnet, dass** der Durchgang (17) und der Spalt (16) im Wesentlichen in einer zur Achse (X-X) der Nabe (11) radialen Richtung ausgerichtet liegen.

6. Anordnung nach Anspruch 4, **dadurch gekennzeichnet, dass** die Ränder des Durchgangs (17') um mindestens 30° in Bezug auf eine zur Achse (X-X) der Nabe radiale Richtung geneigt sind.

7. Anordnung nach Anspruch 3, **dadurch gekennzeichnet, dass** der Kranz (12") über seinen gesamten Umfang ununterbrochen ist.

8. Anordnung nach Anspruch 1, **dadurch gekennzeichnet, dass** eine der Speichen (113) eine seitliche Öffnung (119) zur Aufnahme des Führungsdorns (30) begrenzt, wobei die Öffnung die äußere Umfangsfläche des Kranzes (112) und den Spalt (116) verbindet.

9. Anordnung nach Anspruch 1, **dadurch gekennzeichnet, dass** der Fräskörper (212, 213) sich nur über einen Umfangsbereich der Nabe (211) erstreckt, wobei der Spalt (216) außerhalb desselben liegt.

10. Anordnung nach einem beliebigen der vorhergehenden Ansprüche, **dadurch gekennzeichnet, dass** die Fräsvorrichtung (1) außerdem ein Mittel (20) für den Antrieb der Fräse (10; 110; 210) aufweist, das geeignet ist, gleichzeitig zum Zwecke des Antriebs der Fräse um die Achse (X-X) ihrer Nabe um den Führungsdorn (30) herum in Eingriff zu treten und mit der Nabe (11; 111; 211) gekoppelt zu werden.

## Claims

1. A system for bone preparation, in particular glenoid preparation,
comprising:
- an orthopedic guide pin (30) having a distal end portion that is designed to be introduced into a bone material, and
- an orthopedic milling machine (1) having a burr (10; 110; 210) that includes a hub (11; 111; 211) and a milling body (12, 13; 112, 113; 212, 213), which is provided with bone etching reliefs (14; 114; 214);
said hub being provided with an axial bore (15; 115; 215) for receiving the pin (30), said bore emerging on the respectively proximal and distal, axially opposed faces of the hub;
said hub delimiting a lateral passage slot (16; 116; 216) for the guide pin (30), said slot emerging substantially radially in the bore and connecting the proximal and distal faces of the hub; and
said milling body extending transversely to the hub, while outwardly surrounding the periphery of the hub at least in part, while leaving the opening of the slot open, radially opposite the bore,
**characterized in that** the milling body (12, 13; 112, 113; 212, 213) includes both a crown (12; 12"; 112) or portion of crown (212), radially distant from the hub, and lugs (13; 113; 213) that connect the hub and the crown or portion of crown in a substantially radial direction and that are distributed around the hub (11; 111; 211),
and **in that** the hub (11; 111; 211) is further designed, while the distal end portion of the pin (30) is placed into a bone material, to be laterally approached to the pin at an end, emerging from the bone material, of this distal end portion so that this end of the distal end portion of the pin is engaged radially through the slot (16; 116; 216) until reaching the inside of the bore (15; 115, 215).

2. The system according to claim 1, **characterized in that** the milling body (12, 13; 112, 113) extends all around the hub (11; 111),

3. The system according to claim 2, **characterized in that** the slot (16) emerges, radially opposite the bore (15), in a free volume (V) delimited between two consecutive lugs (13) around the hub (11),

4. The system according to claim 3, **characterized in that** the crown delimits a lateral passage (17; 17') for the guide pin (30), said passage connecting said free volume (V) and the outer peripheral surface of the crown.

5. The system according to claim 4, **characterized in that** the passage (17) and the slot (16) are situated substantially aligned in a direction radial to the axis (X-X) of the hub (11).

6. The system according to claim 4, **characterized in that** the edges of the passage (17') are inclined by at least 30° relative to a direction radial to axis X-X of the hub.

7. The system according to claim 3, **characterized in that** the crown (12") is uninterrupted around the entire periphery thereof.

8. The system according to claim 1, **characterized in that** one of the lugs (113) delimits an opening (119) for laterally receiving the guide pin (30), said opening connecting the outer peripheral surface of the crown (112) and the slot (116).

9. The system according to claim 1, **characterized in that** the milling body (212, 213) extends over only a peripheral portion of the hub (211), outside which the slot (216) is situated.

10. The system according to any one of the preceding claims, **characterized in that** the milling machine (1) also includes means (20) for driving the burr (10; 110; 210), which are adapted to be simultaneously engaged around the guide pin (30) and coupled to the hub (11; 111; 211) in order to drive the burr around the axis (X-X) of its hub.
